# EUROPEAN PATENT APPLICATION

(11) **EP 4 378 336 A1**
(43) Date of publication of application: **05.06.2024**
(21) Application number: 24152966.8
(22) Date of filing: 06.07.2022
(51) Int. Cl.: A24F 40/485, A24F 40/10, A24F 40/30

(54) **AEROSOL PROVISION SYSTEM**

(30) Priority: 19.07.2021 GB 202110331; 19.07.2021 GB 202110326; 19.07.2021 GB 202110328; 19.07.2021 GB 202110330
(62) Divisional of application: 22743861.1
(71) Applicant: Nicoventures Trading Limited, London WC2R 3LA (GB)
(72) Inventor: POTTER, Mark, LONDON, WC2R 3LA (GB)
(74) Representative: D Young & Co LLP

(57) **Abstract**

An aerosol provision system 1 comprising a first reservoir 12 for containing an aerosolisable material for vaporising, and a vaporiser 14, comprising a heating element 22, for vaporising the aerosolisable material from the first reservoir 12. There may be also provided a second reservoir 102, comprising flavouring material, configured to receive vaporised aerosolisable material from the vaporiser 14. The flavouring material may be then configured to be at least partially heated from vaporised aerosolisable material from the vaporiser 14 which flows through the second reservoir 102, and may be further configured to be at least partially heated by the heating element 22 from the vaporiser 14. This may thus provide a more consistent form of heat to the second reservoir 102, and may allow for a boost of preheat to the second reservoir 102 which may be advantageous in situations when the aerosol provision system 1 has not been used for a period of sustained inactivity.

## Description

### Field

The present disclosure relates to aerosol provision systems such as, but not limited to, nicotine delivery systems (e.g. electronic cigarettes and the like).

### Background

Electronic aerosol provision systems such as electronic cigarettes (e-cigarettes) generally contain aerosolisable material, such as a reservoir of fluid or liquid containing a formulation, typically but not necessarily including nicotine, or a solid material such as a tobacco-based product, from which a vapour/aerosol is generated for inhalation by a user, for example through heat vaporisation. Thus, an aerosol provision system will typically comprise a vaporiser, e.g. a heating element, arranged to vaporise a portion of aerosolisable material to generate a vapour.

Once a vapour has been generated, the vapour may be passed through flavouring material to add flavour to the vapour, after which the (flavoured) vapour may be then delivered to a user via a mouthpiece or outlet from the aerosol provision system.

It is common for aerosol provision systems to comprise a modular assembly, often having two main functional parts, namely a control unit / body, and disposable / replaceable consumable part. Such a consumable part, in some embodiments, may take the resemblance of a cigarette.

Various approaches are described herein which seek to improve existing aerosol provision systems, such as those outlined above, in a number of different ways.

### Summary

According to a first aspect of certain embodiments there is provided an aerosol provision system comprising:
a first reservoir for containing an aerosolisable material for vaporising;
a vaporiser for vaporising the aerosolisable material from the first reservoir, wherein the vaporiser comprises a heating element, and wherein the vaporiser is tubular;
a second reservoir comprising a flavouring material, wherein the second reservoir is configured to receive vaporised aerosolisable material from the vaporiser;
wherein flavouring material from the second reservoir is configured to be at least partially heated from vaporised aerosolisable material from the vaporiser which flows through the second reservoir, and is configured to be at least partially heated by the heating element from the vaporiser.

It will be appreciated that features and aspects of the invention described above in relation to the various aspects of the invention are equally applicable to, and may be combined with, embodiments of the invention according to other aspects of the invention as appropriate, and not just in the specific combinations described herein.

### Brief Description of the Drawings

Embodiments of the invention will now be described, by way of example only, with reference to the accompanying drawings, in which:
Figure 1 schematically represents a perspective exploded view of an aerosol provision system, comprising a consumable part and a body, in accordance with certain embodiments of the disclosure.
Figure 2 schematically represents a cross-sectional perspective view of a portion of the aerosol provision system from Figure 1, in accordance with certain embodiments of the disclosure.
Figure 3 schematically represents a cross-sectional perspective view which is similar to Figure 2, but which additionally illustrates the flow of air and vapour through the aerosol provision system, in accordance with certain embodiments of the disclosure.

### Detailed Description

Aspects and features of certain examples and embodiments are discussed / described herein. Some aspects and features of certain examples and embodiments may be implemented conventionally and these are not discussed / described in detail in the interests of brevity. It will thus be appreciated that aspects and features of apparatus and methods discussed herein which are not described in detail may be implemented in accordance with any conventional techniques for implementing such aspects and features.

The present disclosure relates to non-combustible aerosol provision systems, which may also be referred to as aerosol provision systems, such as e-cigarettes. According to the present disclosure, a "non-combustible" aerosol provision system is one where a constituent aerosolisable material of the aerosol provision system (or component thereof) is not combusted or burned in order to facilitate delivery to a user. Aerosolisable material, which also may be referred to herein as aerosol generating material or aerosol precursor material, is material that is capable of generating aerosol, for example when heated, irradiated or energized in any other way.

Throughout the following description the term "e-cigarette" or "electronic cigarette" may sometimes be used, but it will be appreciated this term may be used interchangeably with aerosol provision system / device and electronic aerosol provision system / device. An electronic cigarette may also known as a vaping device or electronic nicotine delivery system (END), although it is noted that the presence of nicotine in the aerosolisable material is not a requirement.

In some embodiments, the non-combustible aerosol provision system is a hybrid system to generate aerosol using a combination of aerosolisable materials, one or a plurality of which may be heated. In some embodiments, the hybrid system comprises a liquid or gel aerosolisable material and a solid aerosolisable material. The solid aerosolisable material may comprise, for example, tobacco or a non-tobacco product.

Typically, the non-combustible aerosol provision system may comprise a consumable part and a device / body which is configured to releasably engage with the consumable part.

The aerosol provision system may be provided with a means for powering a vaporiser therein, and there may be provided an aerosolisable material transport element for receiving the aerosolisable material that is to be vaporised. The aerosol provision system may also be provided with a reservoir for containing aerosolisable material, and in some embodiments a further reservoir for containing flavouring material for flavouring a generated vapour from the aerosol provision system.

In some embodiments, the vaporiser may be a heater/heating element capable of interacting with the aerosolisable material so as to release one or more volatiles from the aerosolisable material to form a vapour/aerosol. In some embodiments, the vaporiser is capable of generating an aerosol from the aerosolisable material without heating. For example, the vaporiser may be capable of generating a vapour/aerosol from the aerosolisable material without applying heat thereto, for example via one or more of vibrational, mechanical, pressurisation or electrostatic means.

In some embodiments, the substance to be delivered may be an aerosolisable material which may comprise an active constituent, a carrier constituent and optionally one or more other functional constituents.

The active constituent may comprise one or more physiologically and/or olfactory active constituents which are included in the aerosolisable material in order to achieve a physiological and/or olfactory response in the user. The active constituent may for example be selected from nutraceuticals, nootropics, and psychoactives. The active constituent may be naturally occurring or synthetically obtained. The active constituent may comprise for example nicotine, caffeine, taurine, theine, a vitamin such as B6 or B12 or C, melatonin, a cannabinoid, or a constituent, derivative, or combinations thereof. The active constituent may comprise a constituent, derivative or extract of tobacco or of another botanical. In some embodiments, the active constituent is a physiologically active constituent and may be selected from nicotine, nicotine salts (e.g. nicotine ditartrate/nicotine bitartrate), nicotine-free tobacco substitutes, other alkaloids such as caffeine, or mixtures thereof.

In some embodiments, the active constituent is an olfactory active constituent and may be selected from a "flavour" and/or "flavourant" which, where local regulations permit, may be used to create a desired taste, aroma or other somatosensorial sensation in a product for adult consumers. In some instances such constituents may be referred to as flavours, flavourants, flavouring material, cooling agents, heating agents, and/or sweetening agents. They may include naturally occurring flavour materials, botanicals, extracts of botanicals, synthetically obtained materials, or combinations thereof (e.g., tobacco, cannabis, licorice (liquorice), hydrangea, eugenol, Japanese white bark magnolia leaf, chamomile, fenugreek, clove, maple, matcha, menthol, Japanese mint, aniseed (anise), cinnamon, turmeric, Indian spices, Asian spices, herb, wintergreen, cherry, berry, red berry, cranberry, peach, apple, orange, mango, clementine, lemon, lime, tropical fruit, papaya, rhubarb, grape, durian, dragon fruit, cucumber, blueberry, mulberry, citrus fruits, Drambuie, bourbon, scotch, whiskey, gin, tequila, rum, spearmint, peppermint, lavender, aloe vera, cardamom, celery, cascarilla, nutmeg, sandalwood, bergamot, geranium, khat, naswar, betel, shisha, pine, honey essence, rose oil, vanilla, lemon oil, orange oil, orange blossom, cherry blossom, cassia, caraway, cognac, jasmine, ylang-ylang, sage, fennel, wasabi, piment, ginger, coriander, coffee, hemp, a mint oil from any species of the genus Mentha, eucalyptus, star anise, cocoa, lemongrass, rooibos, flax, ginkgo biloba, hazel, hibiscus, laurel, mate, orange skin, rose, tea such as green tea or black tea, thyme, juniper, elderflower, basil, bay leaves, cumin, oregano, paprika, rosemary, saffron, lemon peel, mint, beefsteak plant, curcuma, cilantro, myrtle, cassis, valerian, pimento, mace, damien, marjoram, olive, lemon balm, lemon basil, chive, carvi, verbena, tarragon, limonene, thymol, camphene), flavour enhancers, bitterness receptor site blockers, sensorial receptor site activators or stimulators, sugars and/or sugar substitutes (e.g., sucralose, acesulfame potassium, aspartame, saccharine, cyclamates, lactose, sucrose, glucose, fructose, sorbitol, or mannitol), and other additives such as charcoal, chlorophyll, minerals, botanicals, or breath freshening agents. They may be imitation, synthetic or natural ingredients or blends thereof. They may be in any suitable form, for example, liquid such as an oil, solid such as a powder, or gasone or more of extracts (e.g., licorice, hydrangea, Japanese white bark magnolia leaf, chamomile, fenugreek, clove, menthol, Japanese mint, aniseed, cinnamon, herb, wintergreen, cherry, berry, peach, apple, Drambuie, bourbon, scotch, whiskey, spearmint, peppermint, lavender, cardamom, celery, cascarilla, nutmeg, sandalwood, bergamot, geranium, honey essence, rose oil, vanilla, lemon oil, orange oil, cassia, caraway, cognac, jasmine, ylang-ylang, sage, fennel, piment, ginger, anise, coriander, coffee, or a mint oil from any species of the genus Mentha), flavour enhancers, bitterness receptor site blockers, sensorial receptor site activators or stimulators, sugars and/or sugar substitutes (e.g., sucralose, acesulfame potassium, aspartame, saccharine, cyclamates, lactose, sucrose, glucose, fructose, sorbitol, or mannitol), and other additives such as charcoal, chlorophyll, minerals, botanicals, or breath freshening agents. They may be imitation, synthetic or natural ingredients or blends thereof. They may be in any suitable form, for example, oil, liquid, or powder.

In some embodiments, the flavouring material (flavour) may comprise menthol, spearmint and/or peppermint. In some embodiments, the flavour comprises flavour components of cucumber, blueberry, citrus fruits and/or redberry. In some embodiments, the flavour comprises eugenol. In some embodiments, the flavour comprises flavour components extracted from tobacco. In some embodiments, the flavour may comprise a sensate, which is intended to achieve a somatosensorial sensation which are usually chemically induced and perceived by the stimulation of the fifth cranial nerve (trigeminal nerve), in addition to or in place of aroma or taste nerves, and these may include agents providing heating, cooling, tingling, numbing effect. A suitable heat effect agent may be, but is not limited to, vanillyl ethyl ether and a suitable cooling agent may be, but not limited to eucalyptol, WS-3.

The carrier constituent may comprise one or more constituents capable of forming an aerosol. In some embodiments, the carrier constituent may comprise one or more of glycerine, glycerol, propylene glycol, diethylene glycol, triethylene glycol, tetraethylene glycol, 1,3-butylene glycol, erythritol, meso-Erythritol, ethyl vanillate, ethyl laurate, a diethyl suberate, triethyl citrate, triacetin, a diacetin mixture, benzyl benzoate, benzyl phenyl acetate, tributyrin, lauryl acetate, lauric acid, myristic acid, and propylene carbonate.

The one or more other functional constituents may comprise one or more of pH regulators, colouring agents, preservatives, binders, fillers, stabilizers, and/or antioxidants.

As noted above, aerosol provision systems (e-cigarettes) often comprise a modular assembly including both a reusable part (body / device) and a replaceable consumable (cartridge) part. Devices conforming to this type of two-part modular configuration may generally be referred to as two-part devices. It is also common for electronic cigarettes to have a generally elongate shape. For the sake of providing a concrete example, certain embodiments of the disclosure described herein comprise this kind of generally elongate two-part device employing consumable parts. However, it will be appreciated the underlying principles described herein may equally be adopted for other electronic cigarette configurations, for example modular devices comprising more than two parts, as devices conforming to other overall shapes, for example based on so-called box-mod high performance devices that typically have a more boxy shape.

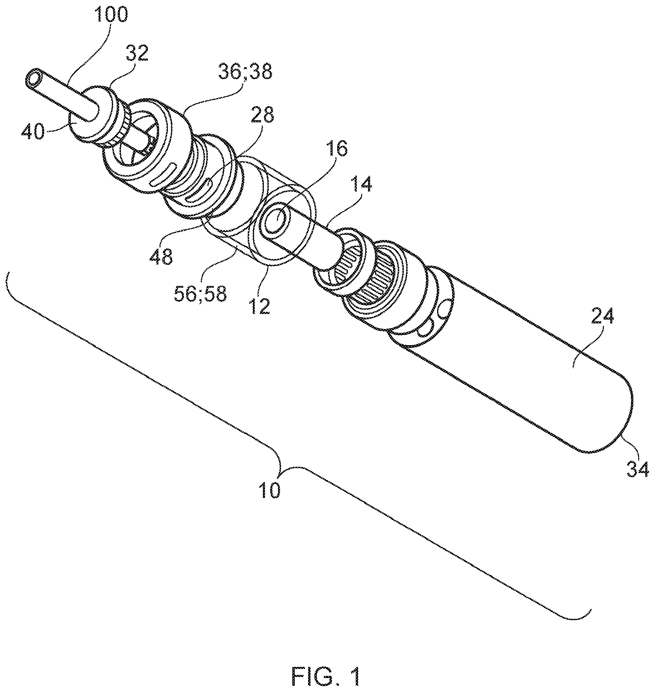

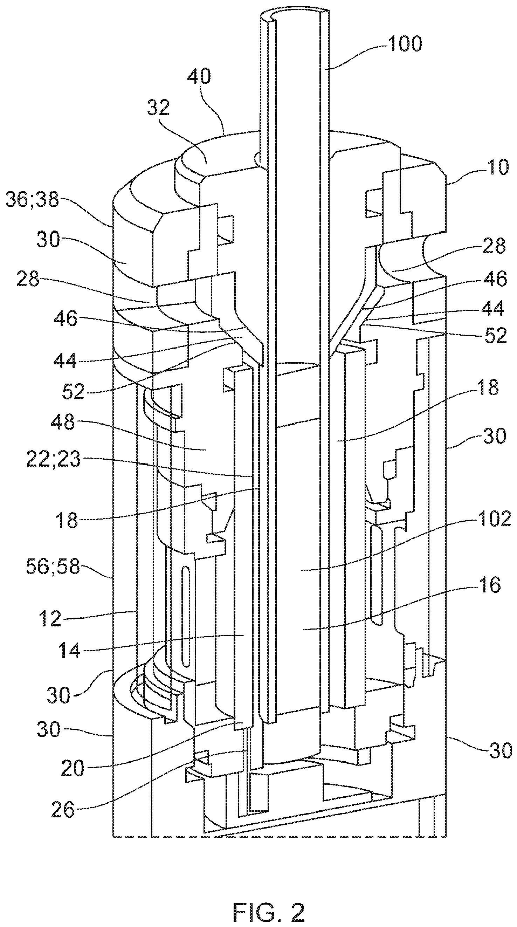

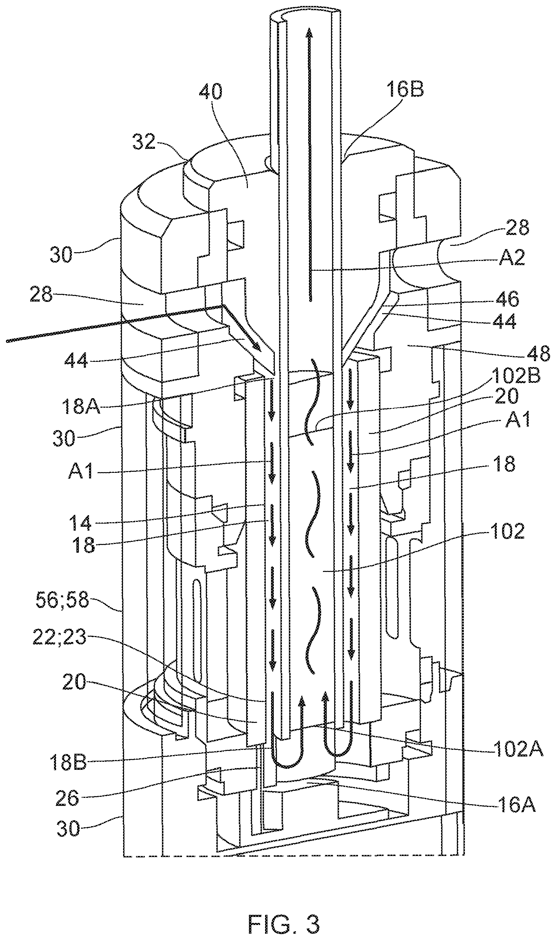

From the forgoing therefore, and with reference to Figures 1-3, in accordance with some embodiments provided herein is an aerosol provision system 1 which may in some embodiments be provided as a body 10 which can releasably accommodate, and/or be releasably attached to, a consumable part 100. The aerosol provision system 1 comprises a first reservoir 12 for containing an aerosolisable material for vaporising, and a vaporiser 14 for vaporising the aerosolisable material from the first reservoir 12. The aerosol provision system 1 also defines a cavity 16 which is configured to receive the consumable part 100.

With respect to the consumable part 100, this may comprise the flavouring material, which may be located in a second reservoir 102.

In accordance with some embodiments, the vaporiser 14 may be tubular and surround the cavity 16, which may provide for a more efficient spacing/layout of the vaporiser 14 with respect to the cavity 16 in such a way that the aerosol provision system 1 can be smaller, and more compact.

Also in accordance with some embodiments, such as the embodiments shown in Figures 1-3, the aerosol provision system 1 may comprise a first channel 18 between the vaporiser 14 and the cavity 16, wherein the vaporiser 14 is configured to impart vaporised aerosolisable material into the first channel 18. In this way, and as will be described, once vaporised aerosolisable material is imparted to the first channel 18 by the vaporiser 14, the vaporised aerosolisable material may then pass through the first channel 18, into the cavity 16 and through any consumable part 100 located therein (including any second reservoir 102 thereof, if present), and finally out of the consumable part 100 through to the user's mouth, for inhalation of the vaporised aerosolisable material by the user.

In accordance with some of the above embodiments, the first channel 18 (where present) may be parallel to the cavity 16, as shown in the embodiments from Figures 1-3. Additionally/alternatively, in accordance with some embodiments, during use of the aerosol provision system 1, air may be configured to flow in a first direction A1 along the first channel 18, and vaporised aerosolisable material from the vaporiser 14 configured to flow in a second direction A2 through the consumable part 100 in the cavity (or, put differently, principally flow in a second direction A2 through the second reservoir 102) wherein the second direction A2 is opposite to the first direction A1. In such embodiments, the first direction A1 may be parallel to a length of the first channel 18, which extends between a first end 18A of the first channel 18 and a second end 18B of the first channel (which may be opposite the first end 18A of the first channel 18). Equally, in accordance with some embodiments, the second direction A2 may be parallel to a length of the cavity 16, which extends between a first end 16A of the cavity 16 (which may be a closed first end 16A in some embodiments) and a second end 16B of the cavity 16 (which may be opposite the first end 16A of the cavity 16, and which in some embodiments may be an open second end 16B). Where the second reservoir 102 is present, in such embodiments, the second direction A2 may in some embodiments thereof be parallel to a length of the second reservoir 102, which extends between a first end 102A of the second reservoir 102 and a second end 102B of the second reservoir 102 (which may be opposite the first end 102A of the second reservoir 102).

In embodiments where the second direction A2 is opposite to the first direction A1, this may allow for a more compact aerosol provision system 1, such as those shown in the embodiments from the Figures, As well, the change in direction from the first direction A1 to the second direction A2 may increase the turbidity of the air passing from the first channel 18 into the cavity 16; the consumable part 100; and/or the second reservoir 102, which may assist with a better passage of vapour through such features along the second direction A2.

Returning to the features of the first channel 18, in accordance with some embodiments, any provided first channel 18 may be tubular, and/or may be located outside of the cavity 16. In such embodiments thereof, the first channel 18 may then extend from the previously described first end 18A and the second end 18B (which may be opposite the first end 18A) for the first channel 18. In accordance with some embodiments, the first channel 18 may be located between the vaporiser 14 and any provided second reservoir 102, and/or may be located between the first reservoir 12 and any provided second reservoir 102, as shown in the embodiments from the Figures.

With respect to the vaporiser 14, as noted previously the vaporiser 14 may, or may not comprise a heater/heating element to form the vaporised aerosolised material. Though in embodiments where the vaporiser 14 comprises a heating element 22, in accordance with some embodiments thereof, the vaporiser 14 may further comprise an aerosolisable material transport element 20 (also called a wick) for delivering aerosolisable material from the first reservoir 12 to the heating element 22. In respect of such embodiments, it will be appreciated that a number of different combinations of aerosolisable material transport element 20 and/or heating element 22 might be employed. For instance, in accordance with some embodiments, the heating element 22 may comprise a conductive material located on an external surface of the aerosolisable material transport element 20, such as a surface 23 which is exposed to the first channel 18. Such conductive material may appreciably take any required shape on the surface of the aerosolisable material transport element 20, e.g. a spiral pattern; a raster pattern; or a zig-zag pattern such to allow the heating element 22 to efficiently vaporise the aerosolisable material in the aerosolisable material transport element 20. As will be appreciated, the heating element 22 (or the vaporiser 14 in general, where a heating element 22 is not employed) may be connected to a power source 24 from the aerosol provision system 1, which in some embodiments may be a battery. In embodiments where the aerosol provision system 1 comprises the body 10 which can releasably accommodate, and/or be releasably attached to, the consumable part 100, in some embodiments thereof the power source 24 may be located in the body 10. Electrical power from the power source 24 may be supplied via a wired connection 26 to the vaporiser 14, and/or appreciably may be delivered to the vaporiser 14 in some embodiments wirelessly or through a non-contact power transmission system between the power source 24 and the vaporiser.

As to the structure and composition of any provided aerosolisable material transport element 20 (wick), it will be appreciated that the aerosolisable material transport element 20 may be anything which can appropriately draw the aerosolisable material to a position where it can be vaporised by the heating element 22 during use of the aerosol provision system 1. In that respect therefore, in accordance with some embodiments, the aerosolisable material transport element 20 may comprise a porous material, and/or a fibrous material or a solid material. In some embodiments, the aerosolisable material transport element 20 may be made of cotton, or could be made of a ceramic material. Thus appreciating the foregoing, and at a general level, it will be appreciated the specific structure and composition of the aerosolisable material transport element 20 may be varied to best cater for any intended application of the aerosol provision system 1.

For delivering air into the aerosol provision system 1, the aerosol provision system 1 may comprise at least one air inlet 28. In some embodiments, a plurality of air inlets 28 may be provided, and in some very particular embodiments (such as those shown in the Figures), the plurality of air inlets 28 may be disposed on side surfaces 30 of the aerosol provision system 1 or body 10, such as in some particular embodiment on side surfaces 30 which extend between a first end 32 of the body 10 and a second end 34 of the body 10 which is opposite the first end 32 of the body 10. By providing more than one air inlet 28, this may conveniently allow for continued operation of the aerosol provision system 1, even if one of the air inlets 28 becomes blocked or inoperable during use. To better ensure an even distribution of air flow through the aerosol provision system 1, in accordance with some embodiments, there may be provided a pair of air inlets 28 located on opposing side surfaces 30 from the aerosol provision system 1 (or body 10), such as in the embodiments shown in the Figures.

To better prevent dirt or fluid from entering each air inlet 28 when the aerosol provision system 1 is not in use, in accordance with some embodiments, there may be provided a closing member 36 which is moveable between a first position in which the at least one air inlet 28 is open and a second position in which the at least one air inlet 28 is blocked. Such a closing member 36 may comprise a variety of different forms, such as a valve for each air inlet; a rotatable sleeve which is rotatable between the first position and the second position; and/or a moveable latch. In a particular embodiment, such as those shown in the Figures, the closing member may comprise a cap 38 which is moveable or rotatable between the first position and the second position. In accordance with some embodiments, the closing member 36 may be configured to make an audible noise (such as a 'click') when the closing member 36 reaches the first position and/or the second position. Such an audible noise may, for instance, be created by the closing member 36 engaging a slot, or ratchet, at each of the first position and the second position. Said slot or ratchet might then be disengaged by the user then applying force to move the closing member 36 from said first or second position towards the other of the first and second positions. With the audible noise, the user will then better know when the closing member 36 is properly located at either the first position and/or the second position.

In accordance with some embodiments, the aerosol provision system 1 (or body 10) may be provided with a retaining member 40, for gripping a consumable part 100 received in the aerosol provision system 1 (or body 10). Such a retaining member 40, which in accordance with some embodiments may be a retaining portion 40 and/or a retaining plug 40, is illustrated in the embodiments from the Figures. The retaining member 40 may surround any provided cavity 16, and may in some particular embodiments surround any second open end 16B of the cavity 16. Thus in embodiments where the retaining member 40 is provided, the retaining member 40 may provide a frictional holding force on the consumable part 100 received in the aerosol provision system 1 (or body 10). Put differently, the retaining member 40 in some embodiments may be configured to releasably accommodate the consumable part 100 received in the aerosol provision system 1 (or body 10). In this way therefore, the retaining member 40 better prevents any consumable part 100 from being accidentally disengaged from, and/or from sliding out of, the aerosol provision system 1 (or body 10) during use, and may better orientate the consumable part 100 in the aerosol provision system 1 (or body 10/cavity 16) during use.

Returning to the at least one air inlet 28, in accordance with some embodiments the aerosol provision system 1 may comprise an initial air channel 44 between the at least one air inlet 28 and the first channel 18. The initial air channel 44 may in be inclined with respect to the first channel 18 and the at least one air inlet 28 for changing the direction of air flowing from the at least one air inlet 28 into the first channel 18. In this way, the initial air channel 44 advantageously provides for a channel which may better guide and prepare the air for entry into the first channel 18. In this way, the presence of the air channel 44 may provide for a more effective flow of air through the first channel 18. As shown in the embodiments from the Figures, any provided initial air channel 44 may in some embodiments be located next to, and feed air into, the first end 18A of the first channel 18.

In embodiments where the retaining member 40 is also employed, in accordance with some embodiments thereof, a surface of the retaining member 40 may define a first surface 46 of the initial air channel 44. In this way, where the retaining member 40 (which may act to better grip and/or orientate the consumable part 100) is removed, this may render the aerosol provision system 1 inoperable, due to air then being able to escape the system through the exposed area where the first surface 46 would otherwise be. In other words, a surface of the retaining member 40 defining the first surface 46 of the initial air channel 44 may act as a safety feature for the aerosol provision system 1. As to the exact shape of any such surface of the retaining member 40 in such embodiments, it will be appreciated that this shape will depend on the relative location of the retaining member 40 to the initial air channel 44. However, in accordance with some embodiments, the retaining member 40 may be annular, and/or the surface of the retaining member may be a conical surface. With this in mind, and for the sake of completeness, it is envisaged in some embodiments that the surface of the retaining member 40 may equally define a surface 46 from another portion of the air channel between the at least one air inlet 28 and the vaporiser 14, to similarly achieve the above noted safety feature.

Returning to aspects of the first channel 18, as noted previously, the first channel 18 may comprise the first end 18A and the second end 18B (which may be opposite the first end 18A), and/or in some embodiments may be cylindrical. In this way, and where the retaining member is employed 40, in some embodiments thereof, the first end 18A of the first channel 18 may be located between the retaining member 40 and the second end 18B of the first channel 18. In this way, and in some embodiments, the first end 18A of the first channel 18 and any open second end 16B of the cavity 16 (which may receive the consumable part 100) may be located on the same side of the second end 18B of the first channel 18. In these embodiments therefore, these may assist with implementing any previously described change in the direction of the air passing through the first channel 18 (e.g. in the direction A1) versus the flow of vapour passing through the cavity 16 and/or the second reservoir 102 (e.g. in the direction A2).

Turning to the first reservoir 12, for better reducing any leakage of aerosolisable material from the first reservoir 12, in accordance with some embodiments the first reservoir 12 may comprise a sealing member 48 for sealing a portion of the first reservoir 12. Conveniently, in some particular embodiments, the sealing member 48 may additionally comprise a surface which defines a second surface 52 of the initial air channel 44. In this way, where the sealing member 48 is removed and/or sufficiently worn out, a leakage path in the initial air channel 44 (or more generally a leakage path in the air path between the at least one air inlet 28 and the vaporiser 14) may be introduced.to effectively render the aerosol provision system 1 inoperable, due to air then being able to escape the system through the exposed area where the second surface 52 would otherwise be. In other words, a surface of the sealing member 48 defining the second surface 52 of the initial air channel 44 may act as a safety feature for the aerosol provision system 1. Again, and for the sake of completeness, it is envisaged in some embodiments that the surface of the sealing member 48 may equally define a surface 52 from another portion of the air channel between the at least one air inlet 28 and the vaporiser 14, to similarly achieve the above noted safety feature.

As to the shape of the first reservoir 12, it will be appreciated that it may take any required shape to supply aerosolisable material to the vaporiser 14 (and/or the aerosolisable material transport element 20, where present). In some embodiments however, such as those shown in the Figures, the first reservoir 12 may be cylindrical, and potentially may extend between a first end and second end. In some particular embodiments thereof where the sealing member 48 is additionally employed, the sealing member 48 may in some cases define at least one of the first end and the second end of the first reservoir 12, as shown in the embodiment of the Figures. Staying with the first reservoir 12, in accordance with some embodiments, the aerosol provision system 1 and/or the first reservoir 12 may comprise a viewing means 56 for allowing a user to observe a quantity or level of aerosolisable material inside the first reservoir 12. Conceivably, such a viewing means 56 may comprise a transparent or translucent portion 58 from the first reservoir 12, or may comprise a window for viewing inside the first reservoir 12. In this way, the viewing means 56 thus allows the user to make a visual determination as to when aerosolisable material in the first reservoir 12 is low/depleted, or may allow the user to identity any malfunctions that might be otherwise viewable inside the first reservoir 12.

Turning to the cavity 16, as noted previously the cavity 16 may in some embodiments extend between a first end 16A of the cavity 16 (which may be a closed first end 16A in some embodiments) and a second end 16B of the cavity 16 (which may be opposite the first end 16A of the cavity 16, and which in some embodiments may be an open second end 16B). The cavity 16 in some embodiments may be cylindrical, such as in embodiments where any provided consumable part 100 is cylindrical and/or where the consumable part 100 resembles the shape of a cigarette. In accordance with some embodiments, the first channel 18 and the cavity 16, and/or the first channel 18 and any provided second reservoir 102, may be separated by at least one partitioning member, such as in some particular embodiments a wall which may be tubular. For the sake of completeness however, it will be appreciated that a function of the at least one partitioning member may be to appropriately separate any vapour passing through any provided second reservoir 102/consumable part 100 from mixing with air flowing through the first channel 18. Accordingly, in some embodiments, an appropriate partition may be achieved by an outer wall of the consumable part 100 acting to suitably separate air in the first channel 18 from vapour passing through the consumable part 100 and/or any second reservoir 102 located therein without the need for any further/separate partitioning member from the aerosol provision system 1 and/or body 10 itself.

Moving to any provided second reservoir 102 from the aerosol provision system 1, for the sake of completeness, the second reservoir 102 could be located in a consumable part 100 which may be releasably accommodated in the aerosol provision system 1, or may be provided in an aerosol provision system 1 where no such consumable part 100 is employed. In such latter embodiments, the second reservoir 102 may for instance be located in (or integrated in) the cavity 16 in a way which inhibits vapour which passes through the second reservoir 102, towards an outlet for inhalation by the user, from mixing with the air passing through the first channel 18. Separation of the air flow from the first channel 18 and the vapour flow through the second reservoir 102 may, in accordance with some embodiments thereof, be achieved by the presence of the at least one partitioning member as described above.

As recited previously, any provided second reservoir 102 from the aerosol provision system 1 may comprise a flavouring material(s), and is configured to receive vaporised aerosolisable material from the vaporiser 14. The flavouring material(s) may comprise any of the previously described flavouring materials, such as (but not limited to) tobacco and/or nicotine. Where the vaporiser 14 comprises the heating element 22, the flavouring material from the second reservoir 102 may be configured to be at least partially heated from vaporised aerosolisable material from the vaporiser 14 which flows through the second reservoir 102. In this way, the heat from the vaporised aerosolisable material may better cause the flavouring material to be imparted into the vaporised aerosolisable material to form the flavoured vapour which is imparted to the user. In this respect, in some particular embodiments, the flavouring material from the second reservoir 102 may be additionally configured to be at least partially heated by the heating element 22 from the vaporiser 14, as shown in the embodiments from the Figures. In this way, heat from the heating element 22 may be transmitted to the second reservoir 102 to provide an additional source of heating to the flavouring material in the second reservoir 102. Such additional heating may be particularly effective when the aerosol provision system 1 is used after a period of sustained inactivity, in so far as the heat from the heating element 22 may then provide an initial amount of heat to the flavouring material in the second reservoir 102 prior to any vaporised aerosolisable material reaching the (otherwise cold) flavouring material in the second reservoir 102. In this way therefore, having heat from the heating element 22 transmitted to the second reservoir 102 to provide an additional source of heating to the flavouring material in the second reservoir 102 may provide a more consistent form of heat to the second reservoir 102, and may allow for a boost of preheat to the second reservoir 102 which may be advantageous in situations when the aerosol provision system 1 has not been used for a period of sustained inactivity.

Conveniently as well, in accordance with some of these embodiments where heat is supplied from the heating element 22, the need for a second heating element, which is separate from the heating element 22, for heating the second reservoir 102 may be potentially removed. Put differently, in accordance with some of these potential embodiments, the aerosol provision system 1 may be provided with only one heating element 22, and/or may be configured such that the second reservoir 102 does not comprise a heating element (for heating the second reservoir 102) which is separate from the heating element 22. Phrased yet another way, in accordance with some of these embodiments, the flavouring material from the second reservoir may be configured to be exclusively heated by both the heat from the vaporised aerosolisable material from the vaporiser 14 which flows through the second reservoir, and by the heat from the heating element 22 from the vaporiser 14.

In the above respect therefore, and in accordance with some embodiments provided herein, there may be provided an aerosol provision system 1 comprising: the first reservoir 12 for containing an aerosolisable material for vaporising; the vaporiser 14 for vaporising the aerosolisable material from the first reservoir 12, wherein the vaporiser 14 comprises a heating element 22; the first channel 18 for receiving aerosolisable from the vaporiser 14; the second reservoir 102 comprising a flavouring material, wherein the second reservoir 102 is configured to receive vaporised aerosolisable material from the vaporiser 14; wherein flavouring material from the second reservoir 102 is configured to be at least partially heated from vaporised aerosolisable material from the vaporiser 14 which flows through the second reservoir 102, and is configured to be at least partially heated by the heating element 22 from the vaporiser 14.

From the foregoing, described herein are a number of different improvements pertaining to a number of a different aspects of aerosol provision systems. In that respect therefore, and at least, there has been described an aerosol provision system comprising:
a first reservoir for containing an aerosolisable material for vaporising;
a vaporiser for vaporising the aerosolisable material from the first reservoir, wherein the vaporiser is tubular and surrounds a cavity from the aerosol provision system;
wherein the cavity is configured to receive a consumable part comprising a flavouring material.

There has also been described an aerosol provision system comprising:
a first reservoir for containing an aerosolisable material for vaporising;
a vaporiser for vaporising the aerosolisable material from the first reservoir, wherein the vaporiser is tubular and surrounds a cavity from the aerosol provision system;
wherein the cavity accommodates a second reservoir comprising a flavouring material, wherein the second reservoir is configured to receive vaporised aerosolisable material from the vaporiser.

There has also been described an aerosol provision system comprising:
a first, cylindrical, reservoir for containing an aerosolisable material for vaporising;
a vaporiser for vaporising the aerosolisable material from the first reservoir;
a first channel for receiving vaporised aerosolisable material from the vaporiser;
a second, cylindrical, reservoir comprising a flavouring material, wherein the second reservoir is configured to receive vaporised aerosolisable material from the first channel;
wherein air is configured to flow in a first direction along the first channel, and vaporised aerosolisable material from the vaporiser is configured to flow in a second direction through the second reservoir, wherein the second direction is opposite to the first direction.

There has also been described an aerosol provision system comprising:
at least one air inlet for delivering air into the aerosol provision system;
a first reservoir for containing an aerosolisable material for vaporising;
a vaporiser for vaporising the aerosolisable material from the first reservoir;
a first channel for receiving vaporised aerosolisable material from the vaporiser, and for receiving air from the at least one air inlet;
an initial air channel between the at least one air inlet and the first channel; and
a retaining member, for gripping a consumable part receivable in the aerosol provision system, wherein a surface of the retaining member defines a first surface of the initial air channel.

There has also been described an aerosol provision system comprising:
a first reservoir for containing an aerosolisable material for vaporising; and
a retaining member, for gripping a consumable part receivable in the aerosol provision system;
wherein the aerosol provision system is configured to inhibit the formation of a vapour in at least one of the aerosol provision system and the consumable part, using aerosolisable material from the first reservoir, when the retaining member is removed.

There has also been described an aerosol provision system comprising:
a first reservoir for containing an aerosolisable material for vaporising;
a vaporiser for vaporising the aerosolisable material from the first reservoir, wherein the vaporiser comprises a heating element, and wherein the vaporiser is tubular;
a second reservoir comprising a flavouring material, wherein the second reservoir is configured to receive vaporised aerosolisable material from the vaporiser;
wherein flavouring material from the second reservoir is configured to be at least partially heated from vaporised aerosolisable material from the vaporiser which flows through the second reservoir, and is configured to be at least partially heated by the heating element from the vaporiser.

There has also been described an aerosol provision system 1 comprising a first reservoir 12 for containing an aerosolisable material for vaporising, and a vaporiser 14, comprising a heating element 22, for vaporising the aerosolisable material from the first reservoir 12. There may be also provided a second reservoir 102, comprising flavouring material, configured to receive vaporised aerosolisable material from the vaporiser 14. The flavouring material may be then configured to be at least partially heated from vaporised aerosolisable material from the vaporiser 14 which flows through the second reservoir 102, and may be further configured to be at least partially heated by the heating element 22 from the vaporiser 14. This may thus provide a more consistent form of heat to the second reservoir 102, and may allow for a boost of preheat to the second reservoir 102 which may be advantageous in situations when the aerosol provision system 1 has not been used for a period of sustained inactivity.

There has also been described an aerosol provision system 1 comprising a first reservoir 12 for containing an aerosolisable material for vaporising, and a vaporiser 14 for vaporising the aerosolisable material from the first reservoir 12. The vaporiser 14 may be tubular and surround a cavity 16 from the aerosol provision system 1, wherein the cavity 16 is configured to receive a consumable part 100 comprising a flavouring material. In this way, a space saving arrangement may be provided. As well, and when the vaporiser 14 comprises a heating element 22, this may beneficially allow the flavouring material to be at least partially heated from vaporised aerosolisable material from the vaporiser 14, and also at least partially heated by the heating element 22. There may also be provided a retaining member 40, for gripping the consumable part 100, wherein a surface of the retaining member defines a first surface 46 of an initial air channel 44 for better directing air flow though the aerosol provision system 1.

There has also been described an aerosol provision system 1 comprising a first, cylindrical, reservoir 12 for containing an aerosolisable material for vaporising, and a vaporiser 14 for vaporising the aerosolisable material from the first reservoir 12. The aerosol provision system 1 may further comprise a first channel 18 for receiving vaporised aerosolisable material from the vaporiser 14, and a second, cylindrical, reservoir 102 comprising a flavouring material. The second reservoir 102 may be configured to receive vaporised aerosolisable material from the first channel 18. Air may be then configured to flow in a first direction A1 along the first channel 18, and vaporised aerosolisable material from the vaporiser 14 configured to flow in a second direction A2 through the second reservoir 102, wherein the second direction A2 is opposite to the first direction A1. In this way a more compact aerosol provision system 1 may be provided.

There has also been described an aerosol provision system 1 comprising a first reservoir 12 for containing an aerosolisable material for vaporising, and a vaporiser 14 for vaporising the aerosolisable material from the first reservoir 12. The aerosol provision system 1 may further comprise at least one air inlet 28 for delivering air into the aerosol provision system 1, and a first channel 18 for receiving vaporised aerosolisable material from the vaporiser 14, and for receiving air from the at least one air inlet 28. An initial air channel 44 may be provided between the at least one air inlet 28 and the first channel 18, along with a retaining member 40, for gripping a consumable part 100 receivable in the aerosol provision system 1, wherein a surface of the retaining member 40 defines a first surface 46 of the initial air channel 44. In this way, where the retaining member 40 is removed, this may render the aerosol provision system 1 inoperable, due to air then being able to escape the system 1 through the exposed area where the first surface 46 would otherwise be.

Appreciating the various disclosure herein, it will also be appreciated that herein has also been disclosed the following embodiments of an aerosol provision system:
1. An aerosol provision system comprising:
   a first reservoir for containing an aerosolisable material for vaporising; and
   a vaporiser for vaporising the aerosolisable material from the first reservoir.
2. An aerosol provision system according to clause 1, wherein the vaporiser comprises a heating element.
3. An aerosol provision system according to clause 1 or 2, wherein the aerosol provision system further comprises a second reservoir.
4. An aerosol provision system according to clause 3, wherein the second reservoir comprises a flavouring material.
5. An aerosol provision system according to clause 3 or 4, wherein the second reservoir is configured to receive vaporised aerosolisable material from the vaporiser.
6. An aerosol provision system according to clause 5, when further dependent on clauses 2 and 4, wherein flavouring material from the second reservoir is configured to be at least partially heated from vaporised aerosolisable material from the vaporiser which flows through the second reservoir.
7. An aerosol provision system according to clause any of clauses 5-6, when further dependent on clause 2, wherein flavouring material from the second reservoir is configured to be at least partially heated by the heating element from the vaporiser.
8. An aerosol provision system according to any preceding clause, further comprising a cavity.
9. An aerosol provision system according to clause 8, wherein the cavity is for accommodating the first reservoir.
10. An aerosol provision system according to clause 8 or 9, wherein the cavity is for accommodating the second reservoir.
11. An aerosol provision system according to any of clauses 8-10, wherein the cavity is configured to receive a consumable part.
12. An aerosol provision system according to clause 11, when further dependent on clause 9, wherein the cavity is configured to receive a consumable part comprising the first reservoir.
13. An aerosol provision system according to clause 11 or 12, when further dependent on clause 10, wherein the cavity is configured to receive a consumable part comprising the second reservoir.
14. An aerosol provision system according to any preceding clause, wherein the vaporiser comprises a tubular portion, and/or wherein the vaporiser comprises a tubular vaporiser.
15. An aerosol provision system according to clause 14, when further dependent on clause 8, wherein the tubular portion and/or the tubular reservoir surrounds the cavity.
16. An aerosol provision system according to any preceding clause, wherein the aerosol provision system comprises at least one air inlet for delivering air into the aerosol provision system.
17. An aerosol provision system according to clause 16, wherein the at least one air inlet comprises a plurality of air inlets.
18. An aerosol provision system according to clause 17, wherein the plurality of air inlets comprises a pair of air inlets located on opposing side surfaces from the aerosol provision system.
19. An aerosol provision system according to clause 17 or 18, when further dependent on clause 8, wherein the plurality of air inlets surround the cavity.
20. An aerosol provision system according to any of clauses 16-19, wherein the aerosol provision system further comprises a first channel for receiving air from the at least one air inlet.
21. An aerosol provision system according to clause 20, wherein the vaporiser is configured to impart vaporised aerosolisable material into the first channel.
22. An aerosol provision system according to clause 20 or 21, when further dependent on clause 8, wherein the first channel is parallel to the cavity.
23. An aerosol provision system according to any of clauses 20-22, when further dependent on clause 8, wherein the first channel is located outside of the cavity.
24. An aerosol provision system according to any of clauses 20-23, when further dependent on clause 11, wherein air is configured to flow in a first direction along the first channel, and vaporised aerosolisable material from the vaporiser is configured to flow in a second direction through a consumable part in the cavity, wherein the second direction is opposite to the first direction.
25. An aerosol provision system according to clause 24, wherein the first channel and the cavity are separated by a partitioning member.
26. An aerosol provision system according to clause 25, wherein the partitioning member is cylindrical.
27. An aerosol provision system according to any preceding clause, when further dependent on clause 8 at least, wherein the cavity is cylindrical; and comprises a first, closed, end; and comprises a second, open, end opposite the first end.
28. An aerosol provision system according to any preceding clause, when further dependent on clause 16 at least, wherein the aerosol provision system further comprises a cap which is moveable between a first position in which the at least one air inlet is open and a second position in which the at least one air inlet is blocked.
29. An aerosol provision system according to clause 28, wherein the cap is rotatable between the first position and the second position.
30. An aerosol provision system according to any preceding clause, when further dependent on clause 20 at least, further comprising an initial air channel between the at least one air inlet and the first channel.
31. An aerosol provision system according to clause 30, wherein the initial air channel is inclined with respect to the first channel and the at least one air inlet for changing the direction of air flowing from the at least one air inlet into the first channel.
32. An aerosol provision system according to any preceding clause, when further dependent on clause 11 at least, wherein the aerosol provision system further comprises a retaining portion, for gripping the consumable part received in the aerosol provision system.
33. An aerosol provision system according to clause 32, wherein the retaining portion surrounds the cavity.
34. An aerosol provision system according to clause 32 or 33, wherein the retaining portion at least partially extends into the cavity.
35. An aerosol provision system according to any of clauses 32-34, wherein the retaining portion at least partially extends about a length direction of the cavity.
36. An aerosol provision system according to any of clauses 32-35, when further dependent on clause 26, wherein the retaining portion extends between the first and second ends of the cavity.
37. An aerosol provision system according to any of clauses 32-36, wherein the retaining portion comprises a retaining member or a retaining plug.
38. An aerosol provision system according to any of clauses 32-37, when further dependent on clause 30 at least, wherein a surface of the retaining portion defines a first surface of the initial air channel.
39. An aerosol provision system according to clause 38, wherein the surface of the retaining portion is a conical surface.
40. An aerosol provision system according to any of clauses 32-39, wherein the retaining portion is annular.
41. An aerosol provision system according to any of clauses 32-40, wherein the retaining portion extends around the cavity.
42. An aerosol provision system according to any of clauses 32-41, wherein the retaining portion is configured to extend between a first end and a second end of the consumable part when the cavity is receiving the consumable part.
43. An aerosol provision system according to any of clauses 32-42, wherein the retaining portion is configured to grip an outer surface of the consumable part.
44. An aerosol provision system according to any of clauses 32-43, wherein the retaining portion is configured to grip an outer surface of the consumable part when the cavity receives the consumable part.
45. An aerosol provision system according to any of clauses 32-44, wherein the retaining portion is configured to grip an outer surface from a first side of the consumable part when the cavity receives the consumable part, and is configured to grip an outer surface from a second side of the consumable part when the cavity receives the consumable part, wherein the second side is opposite the first side.
46. An aerosol provision system according to any of clauses 32-45, when further dependent on clause 20 at least, wherein the first channel comprises a first end and second end opposite the first end, wherein the first end of the first channel is located between the retaining portion and the second end of the first channel.
49. An aerosol provision system according to any preceding clause, when further dependent on clause 30 at least, wherein the first reservoir comprises a sealing member for sealing a portion of the first reservoir, wherein the sealing member comprises a surface which defines a second surface of the initial air channel.
50. An aerosol provision system according to any preceding clause, wherein the vaporiser comprises an aerosolisable material transport element for delivering aerosolisable material from the first reservoir to the vaporiser.
51. An aerosol provision system according to any preceding clause, wherein the first reservoir is cylindrical.
52. An aerosol provision system according to any preceding clause, when further dependent on clause 3 at least, wherein the second reservoir is cylindrical.
53. An aerosol provision system according to any preceding claim, wherein the first reservoir comprises a transparent or translucent portion for allowing a user to observe a quantity of aerosolisable material inside the first reservoir.
54. An aerosol provision system according to any preceding clause, when further dependent on clause 11, wherein the consumable part comprises at least one of tobacco and/or nicotine.
55. An aerosol provision system according to any preceding clause, when the first reservoir comprises at least one of tobacco and/or nicotine.
56. An aerosol provision system according to any preceding clause, when further dependent on clause 3, wherein the second reservoir comprises at least one of tobacco and/or nicotine.
57. An aerosol provision system according to any preceding clause, when further dependent on clause 11, wherein the consumable part is cylindrical.
58. An aerosol provision system according to any preceding clause, when further dependent on clause 11, wherein the consumable part is configured to at least partially project from the cavity when the cavity receives the consumable part.
59. An aerosol provision system according to any preceding clause, when further dependent on clause 11, wherein the consumable part is longer than the cavity.
60. An aerosol provision system according to any preceding clause, when further dependent on clauses 8 and 20 at least, wherein the first channel surrounds the cavity.

For completeness, it may be appreciated that the features from any of the above clauses may further comprise, and/or be combined with, any other feature(s) or combination of feature(s) as herein described, and/or as described with reference to the claims below.

In order to address various issues and advance the art, this disclosure shows by way of illustration various embodiments in which the claimed invention(s) may be practiced. The advantages and features of the disclosure are of a representative sample of embodiments only, and are not exhaustive and/or exclusive. They are presented only to assist in understanding and to teach the claimed invention(s). It is to be understood that advantages, embodiments, examples, functions, features, structures, and/or other aspects of the disclosure are not to be considered limitations on the disclosure as defined by the claims or limitations on equivalents to the claims, and that other embodiments may be utilised and modifications may be made without departing from the scope of the claims. Various embodiments may suitably comprise, consist of, or consist essentially of, various combinations of the disclosed elements, components, features, parts, steps, means, etc. other than those specifically described herein, and it will thus be appreciated that features of the dependent claims may be combined with features of the independent claims in combinations other than those explicitly set out in the claims. The disclosure may include other inventions not presently claimed, but which may be claimed in future.

For instance, although the present disclosure has been described with reference to a "liquid" or "fluid" in the first reservoir, it will be appreciated that this liquid or fluid may be replaced with any aerosolisable material. Equally, where an aerosolisable material is used, it will be appreciated that in some embodiments this aerosolisable material may comprise a liquid or fluid.

With respect to the vaporiser 14, although the vaporiser 14 has been described in some embodiments as comprising a heater/heating element 22, it will be appreciated that in some other embodiments the vaporiser 14 may be capable of generating a vapour/aerosol from the aerosolisable material without applying heat thereto, for example via one or more of vibrational, mechanical, pressurisation or electrostatic means as described previously.

Concerning any provided flavouring material as well, for the sake of completeness it is to be noted that any provided flavouring material need not necessarily be heated to release its flavouring to the vaporised aerosolisable material. In that respect therefore, it is not necessarily the case that any vaporiser 14 in these instances must necessarily require the heating element 22.

Finally as well, whilst a number of the features herein described have been described with reference to embodiments of aerosol provision system 1 which are provided as part of a body 10 which can releasably accommodate, and/or be releasably attached to, a consumable part 100, it will be appreciated that in some embodiments, any of the herein described features from the consumable part 100 and/or the body 10 may be integrated into an aerosol provision system 1 which does not comprise such a consumable part/body 100 configuration.

### FIRST SET OF CLAUSES

1. An aerosol provision system comprising:
   a first reservoir for containing an aerosolisable material for vaporising;
   a vaporiser for vaporising the aerosolisable material from the first reservoir, wherein the vaporiser is tubular and surrounds a cavity from the aerosol provision system;
   wherein the cavity is configured to receive a consumable part comprising a flavouring material.
2. An aerosol provision system according to any preceding clause, wherein the aerosol provision system comprises a first channel between the vaporiser and the cavity, wherein the vaporiser is configured to impart vaporised aerosolisable material into the first channel.
3. An aerosol provision system according to clause 2, wherein the first channel is parallel to the cavity.
4. An aerosol provision system according to any of clauses 2-3, wherein the first channel is located outside of the cavity.
5. An aerosol provision system according to any of clauses 2-4, wherein air is configured to flow in a first direction along the first channel, and vaporised aerosolisable material from the vaporiser is configured to flow in a second direction through a consumable part in the cavity, wherein the second direction is opposite to the first direction.
6. An aerosol provision system according to any of clauses 2-5, wherein the first channel and the cavity are separated by a partitioning member.
7. An aerosol provision system according to any preceding clause, wherein the aerosol provision system comprises at least one air inlet for delivering air into the aerosol provision system.
8. An aerosol provision system according to clause 7, wherein the at least one air inlet comprises a plurality of air inlets.
9. An aerosol provision system according to clause 8, wherein the plurality of air inlets comprises a pair of air inlets located on opposing side surfaces from the aerosol provision system.
10. An aerosol provision system according to any of clauses 7-9, wherein the aerosol provision system further comprises a cap which is moveable between a first position in which the at least one air inlet is open and a second position in which the at least one air inlet is blocked.
11. An aerosol provision system according to clause 10, wherein the cap is rotatable between the first position and the second position.
12. An aerosol provision system according to any of clauses 7-11, when further dependent on clause 2, further comprising an initial air channel between the at least one air inlet and the first channel, wherein the initial air channel is inclined with respect to the first channel and the at least one air inlet for changing the direction of air flowing from the at least one air inlet into the first channel.
13. An aerosol provision system according to clause 12, further comprising a retaining member, for gripping a consumable part received in the aerosol provision system, wherein the retaining member surrounds the cavity, and wherein a surface of the retaining member defines a first surface of the initial air channel.
14. An aerosol provision system according to clause 13, wherein the retaining member is annular, and the surface of the retaining member is a conical surface.
15. An aerosol provision system according to any of clauses 13-14, wherein the first channel comprises a first end and second end opposite the first end, wherein the first end of the first channel is located between the retaining member and the second end of the first channel.
16. An aerosol provision system according to any of clauses 12-15, wherein the first reservoir comprises a sealing member for sealing a portion of the first reservoir, wherein the sealing member comprises a surface which defines a second surface of the initial air channel.
17. An aerosol provision system according to any preceding clause, wherein the vaporiser comprises a heating element.
18. An aerosol provision system according to clause 17, wherein the vaporiser comprises an aerosolisable material transport element for delivering aerosolisable material from the first reservoir to the heating element.
19. An aerosol provision system according to any preceding clause, wherein the first reservoir is cylindrical.
20. An aerosol provision system according to any preceding clause, wherein the cavity is cylindrical; and comprises a first, closed, end; and comprises a second, open, end opposite the first end.
21. An aerosol provision system according to any preceding clause, wherein the first reservoir comprises a transparent or translucent portion for allowing a user to observe a quantity of aerosolisable material inside the first reservoir.
22. An aerosol provision system according to any preceding clause, further comprising the consumable part, wherein the consumable part comprises a second reservoir for containing flavouring material, wherein the second reservoir is configured to receive vaporised aerosolisable material from the vaporiser.
23. An aerosol provision system according to clause 22, when further dependent on clause 17, wherein flavouring material from the second reservoir is configured to be at least partially heated from vaporised aerosolisable material from the vaporiser which flows through the second reservoir, and is configured to be at least partially heated by the heating element from the vaporiser.
24. An aerosol provision system according to any of clauses 22-23, wherein the flavouring material comprises at least one of tobacco and/or nicotine.
25. An aerosol provision system comprising:
   a first reservoir for containing an aerosolisable material for vaporising;
   a vaporiser for vaporising the aerosolisable material from the first reservoir, wherein the vaporiser is tubular and surrounds a cavity from the aerosol provision system;
   wherein the cavity accommodates a second reservoir comprising a flavouring material, wherein the second reservoir is configured to receive vaporised aerosolisable material from the vaporiser.

### SECOND SET OF CLAUSES

1. An aerosol provision system comprising:
   a first, cylindrical, reservoir for containing an aerosolisable material for vaporising;
   a vaporiser for vaporising the aerosolisable material from the first reservoir;
   a first channel for receiving vaporised aerosolisable material from the vaporiser;
   a second, cylindrical, reservoir comprising a flavouring material, wherein the second reservoir is configured to receive vaporised aerosolisable material from the first channel;
   wherein air is configured to flow in a first direction along the first channel, and vaporised aerosolisable material from the vaporiser is configured to flow in a second direction through the second reservoir, wherein the second direction is opposite to the first direction.
2. An aerosol provision system according to clause 1, further comprising a cavity which is configured to accommodate the second reservoir.
3. An aerosol provision system according to clause 2, wherein the first channel is between the vaporiser and the cavity, wherein the vaporiser is configured to impart vaporised aerosolisable material into the first channel.
4. An aerosol provision system according to clause 2 or 3, wherein the first channel is parallel to the cavity.
5. An aerosol provision system according to any of clauses 2-4, wherein the first channel is located outside of the cavity.
6. An aerosol provision system according to any of clauses 2-5, wherein the first channel and the cavity are separated by a partitioning member.
7. An aerosol provision system according to any of clauses 2-6, wherein the cavity is configured to receive a consumable part comprising the second reservoir.
8. An aerosol provision system according to any of clauses 2-7, wherein the vaporiser surrounds the cavity.
9. An aerosol provision system according to any of clauses 2-8, wherein the cavity is cylindrical; and comprises a first, closed, end; and comprises a second, open, end opposite the first end.
10. An aerosol provision system according to any preceding clause, wherein the vaporiser is tubular.
11. An aerosol provision system according to any preceding clause, wherein the aerosol provision system comprises at least one air inlet for delivering air into the aerosol provision system.
12. An aerosol provision system according to clause 11, wherein the at least one air inlet comprises a plurality of air inlets.
13. An aerosol provision system according to clause 12, wherein the plurality of air inlets comprises a pair of air inlets located on opposing side surfaces from the aerosol provision system.
14. An aerosol provision system according to any of clauses 11-13, wherein the aerosol provision system further comprises a cap which is moveable between a first position in which the at least one air inlet is open and a second position in which the at least one air inlet is blocked.
15. An aerosol provision system according to clause 14, wherein the cap is rotatable between the first position and the second position.
16. An aerosol provision system according to any of clauses 11-15, further comprising an initial air channel between the at least one air inlet and the first channel, wherein the initial air channel is inclined with respect to the first channel and the at least one air inlet for changing the direction of air flowing from the at least one air inlet into the first channel.
17. An aerosol provision system according to clause 16, when further dependent on clause 2, further comprising a retaining member, for gripping a consumable part received in the aerosol provision system, wherein the retaining member surrounds the cavity, and wherein a surface of the retaining member defines a first surface of the initial air channel.
18. An aerosol provision system according to clause 17, wherein the retaining member is annular, and the surface of the retaining member is a conical surface.
19. An aerosol provision system according to any of clauses 17-18, wherein the first channel comprises a first end and second end opposite the first end, wherein the first end of the first channel is located between the retaining member and the second end of the first channel.
20. An aerosol provision system according to any of clauses 16-19, wherein the first reservoir comprises a sealing member for sealing a portion of the first reservoir, wherein the sealing member comprises a surface which defines a second surface of the initial air channel.
21. An aerosol provision system according to any preceding clause, wherein the vaporiser comprises a heating element.
22. An aerosol provision system according to clause 21, wherein flavouring material from the second reservoir is configured to be at least partially heated from vaporised aerosolisable material from the vaporiser which flows through the second reservoir, and is configured to be at least partially heated by the heating element from the vaporiser.
23. An aerosol provision system according to clause 21 or 22, wherein the vaporiser comprises an aerosolisable material transport element for delivering aerosolisable material from the first reservoir to the heating element.
24. An aerosol provision system according to any preceding clause, wherein the first reservoir comprises a transparent or translucent portion for allowing a user to observe a quantity of aerosolisable material inside the first reservoir.
25. An aerosol provision system according to any preceding clause, further comprising a consumable part, wherein the consumable part comprises the second reservoir for containing flavouring material, wherein the second reservoir is configured to receive vaporised aerosolisable material from the vaporiser.
26. An aerosol provision system according to any preceding clause, wherein the flavouring material comprises at least one of tobacco and/or nicotine.

### THIRD SET OF CLAUSES

1. An aerosol provision system comprising:
   at least one air inlet for delivering air into the aerosol provision system;
   a first reservoir for containing an aerosolisable material for vaporising;
   a vaporiser for vaporising the aerosolisable material from the first reservoir;
   a first channel for receiving vaporised aerosolisable material from the vaporiser, and for receiving air from the at least one air inlet;
   an initial air channel between the at least one air inlet and the first channel; and
   a retaining member, for gripping a consumable part receivable in the aerosol provision system, wherein a surface of the retaining member defines a first surface of the initial air channel.
2. An aerosol provision system according to clause 1, wherein the retaining member is annular, and the surface of the retaining member is a conical surface.
3. An aerosol provision system according to any preceding clause, wherein the first channel comprises a first end and second end opposite the first end, wherein the first end of the first channel is located between the retaining member and the second end of the first channel.
4. An aerosol provision system according to any preceding clause, wherein the first reservoir comprises a sealing member for sealing a portion of the first reservoir, wherein the sealing member comprises a surface which defines a second surface of the initial air channel.
5. An aerosol provision system according to any preceding clause, further comprising a cavity which is configured to accommodate a second reservoir comprising a flavouring material, wherein the second reservoir is configured to receive vaporised aerosolisable material from the first channel.
6. An aerosol provision system according to clause 5, wherein the cavity is configured to receive the consumable part comprising the second reservoir.
7. An aerosol provision system according to clause 5 or 6, wherein the retaining member surrounds the cavity.
8. An aerosol provision system according to any of clauses 5-7, wherein the second reservoir is cylindrical.
9. An aerosol provision system according to any of clauses 5-8, wherein the vaporiser is tubular and surrounds the cavity.
10. An aerosol provision system according to any of clauses 5-9, wherein the first channel is between the vaporiser and the cavity, wherein the vaporiser is configured to impart vaporised aerosolisable material into the first channel.
11. An aerosol provision system according to any of clauses 5-10, wherein the first channel is parallel to the cavity.
12. An aerosol provision system according to any of clauses 5-11, wherein the first channel is located outside of the cavity.
13. An aerosol provision system according to any of clauses 5-12, wherein air is configured to flow in a first direction along the first channel, and vaporised aerosolisable material from the vaporiser is configured to flow in a second direction through a consumable part in the cavity, wherein the second direction is opposite to the first direction.
14. An aerosol provision system according to any of clauses 5-13, wherein the first channel and the cavity are separated by a partitioning member.
15. An aerosol provision system according to any of clauses 5-14, wherein the cavity is cylindrical; and comprises a first, closed, end; and comprises a second, open, end opposite the first end.
16. An aerosol provision system according to any preceding clause, wherein the at least one air inlet comprises a plurality of air inlets.
17. An aerosol provision system according to clause 16, wherein the plurality of air inlets comprises a pair of air inlets located on opposing side surfaces from the aerosol provision system.
18. An aerosol provision system according to any preceding clause, wherein the aerosol provision system further comprises a cap which is moveable between a first position in which the at least one air inlet is open and a second position in which the at least one air inlet is blocked.
19. An aerosol provision system according to clause 18, wherein the cap is rotatable between the first position and the second position.
20. An aerosol provision system according to any preceding clause, wherein the initial air channel is inclined with respect to the first channel and the at least one air inlet for changing the direction of air flowing from the at least one air inlet into the first channel.
21. An aerosol provision system according to any preceding clause, wherein the vaporiser comprises a heating element.
22. An aerosol provision system according to clause 21, wherein the vaporiser comprises an aerosolisable material transport element for delivering aerosolisable material from the first reservoir to the heating element.
23. An aerosol provision system according to any preceding clause, wherein the first reservoir is cylindrical.
24. An aerosol provision system according to any preceding clause, wherein the first reservoir comprises a transparent or translucent portion for allowing a user to observe a quantity of aerosolisable material inside the first reservoir.
25. An aerosol provision system according to any preceding clause, further comprising the consumable part, wherein the consumable part comprises a second reservoir for containing flavouring material, wherein the second reservoir is configured to receive vaporised aerosolisable material from the vaporiser.
26. An aerosol provision system according to any of clauses 5-15 or 25, when further dependent on clause 21, wherein flavouring material from the second reservoir is configured to be at least partially heated from vaporised aerosolisable material from the vaporiser which flows through the second reservoir, and is configured to be at least partially heated by the heating element from the vaporiser.
27. An aerosol provision system according to any of clauses 5-15 or 25-26, wherein the flavouring material comprises at least one of tobacco and/or nicotine.
28. An aerosol provision system comprising:
   a first reservoir for containing an aerosolisable material for vaporising; and
   a retaining member, for gripping a consumable part receivable in the aerosol provision system;
   wherein the aerosol provision system is configured to inhibit the formation of a vapour in at least one of the aerosol provision system and the consumable part, using aerosolisable material from the first reservoir, when the retaining member is removed.

### REPRESENTATIVE FEATURES

1. An aerosol provision system comprising:
   a first reservoir for containing an aerosolisable material for vaporising;
   a vaporiser for vaporising the aerosolisable material from the first reservoir, wherein the vaporiser comprises a heating element, and wherein the vaporiser is tubular;
   a second reservoir comprising a flavouring material, wherein the second reservoir is configured to receive vaporised aerosolisable material from the vaporiser;
   wherein flavouring material from the second reservoir is configured to be at least partially heated from vaporised aerosolisable material from the vaporiser which flows through the second reservoir, and is configured to be at least partially heated by the heating element from the vaporiser.
2. An aerosol provision system according to clause 1, further comprising a cavity which is configured to accommodate the second reservoir.
3. An aerosol provision system according to clause 2, wherein the cavity is configured to receive a consumable part comprising the second reservoir.
4. An aerosol provision system according to any of clauses 2-3, wherein the vaporiser is tubular and surrounds the cavity.
5. An aerosol provision system according to any of clauses 2-4, wherein the aerosol provision system comprises a first channel between the vaporiser and the cavity, wherein the vaporiser is configured to impart vaporised aerosolisable material into the first channel.
6. An aerosol provision system according to clause 5, wherein the first channel is parallel to the cavity.
7. An aerosol provision system according to any of clauses 5-6, wherein the first channel is located outside of the cavity.
8. An aerosol provision system according to any of clauses 5-7, wherein air is configured to flow in a first direction along the first channel, and vaporised aerosolisable material from the vaporiser is configured to flow in a second direction through a consumable part in the cavity, wherein the second direction is opposite to the first direction.
9. An aerosol provision system according to any of clauses 5-8, wherein the first channel and the cavity are separated by a partitioning member.
10. An aerosol provision system according to any of clauses 5-9, wherein the cavity is cylindrical; and comprises a first, closed, end; and comprises a second, open, end opposite the first end.
11. An aerosol provision system according to any preceding clause, wherein the aerosol provision system comprises at least one air inlet for delivering air into the aerosol provision system.
12. An aerosol provision system according to clause 11, wherein the at least one air inlet comprises a plurality of air inlets.
13. An aerosol provision system according to clause 12, wherein the plurality of air inlets comprises a pair of air inlets located on opposing side surfaces from the aerosol provision system.
14. An aerosol provision system according to any of clauses 11-13, wherein the aerosol provision system further comprises a cap which is moveable between a first position in which the at least one air inlet is open and a second position in which the at least one air inlet is blocked.
15. An aerosol provision system according to clause 14, wherein the cap is rotatable between the first position and the second position.
16. An aerosol provision system according to any of clauses 11-15, when further dependent on clause 5, further comprising an initial air channel between the at least one air inlet and the first channel, wherein the initial air channel is inclined with respect to the first channel and the at least one air inlet for changing the direction of air flowing from the at least one air inlet into the first channel.
17. An aerosol provision system according to clause 16, further comprising a retaining member, for gripping a consumable part received in the aerosol provision system, wherein the retaining member surrounds the cavity, and wherein a surface of the retaining member defines a first surface of the initial air channel.
18. An aerosol provision system according to clause 17, wherein the retaining member is annular, and the surface of the retaining member is a conical surface.
19. An aerosol provision system according to any of clauses 17-18, wherein the first channel comprises a first end and second end opposite the first end, wherein the first end of the first channel is located between the retaining member and the second end of the first channel.
20. An aerosol provision system according to any of clauses 16-19, wherein the first reservoir comprises a sealing member for sealing a portion of the first reservoir, wherein the sealing member comprises a surface which defines a second surface of the initial air channel.
21. An aerosol provision system according to any preceding clause, wherein the vaporiser comprises an aerosolisable material transport element for delivering aerosolisable material from the first reservoir to the heating element.
22. An aerosol provision system according to any preceding clause, wherein the first reservoir is cylindrical.
23. An aerosol provision system according to any preceding clause, wherein the second reservoir is cylindrical.
24. An aerosol provision system according to any preceding clause, wherein the first reservoir comprises a transparent or translucent portion for allowing a user to observe a quantity of aerosolisable material inside the first reservoir.
25. An aerosol provision system according to any preceding clause, further comprising a consumable part, wherein the consumable part comprises the second reservoir.
26. An aerosol provision system according to any preceding clause, wherein the flavouring material comprises at least one of tobacco and/or nicotine.

This divisional application is divided from parent application no. EP22743861.1 (WO2023002153) filed at the EPO on 6 July 2022. This divisional application comprises the full content of the earlier parent application as filed, including the description, claims, abstract, any drawings and any sequence listing. Nevertheless, protection may be sought for any features disclosed herein in combination with any features disclosed in the earlier parent application as filed, which is incorporated herein by reference.

## Claims

1. An aerosol provision system comprising:
a first reservoir for containing an aerosolisable material for vaporising;
a vaporiser for vaporising the aerosolisable material from the first reservoir, wherein the vaporiser comprises a heating element;
an aerosolisable material transport element for delivering aerosolisable material from the first reservoir to the vaporiser;
at least one air inlet for delivering air into the aerosol provision system; and
a cap which is moveable between a first position in which the at least one air inlet is open and a second position in which the at least one air inlet is blocked.

2. An aerosol provision system according to any preceding claim, wherein the cap engages a slot at each of the first position and the second position.

3. An aerosol provision system according to any preceding claim, wherein the cap is configured to make an audible noise when the cap reaches the second position.

4. An aerosol provision system according to any preceding claim, wherein the aerosol provision system further comprises a cavity for receiving vaporised aerosolisable material which is vaporised by the vaporiser.

5. An aerosol provision system according to claim 4, wherein the first reservoir is tubular, and wherein the first reservoir surrounds the cavity.

6. An aerosol provision system according to any of claims 4-5, wherein the cavity is cylindrical; and comprises a first end; and comprises a second, open, end opposite the first end.

7. An aerosol provision system according to any preceding claim, wherein the aerosol provision system further comprises a first channel for receiving air from the at least one air inlet.

8. An aerosol provision system according to claim 7, when further dependent on any of claims 4-6, wherein the first channel is parallel to the cavity.

9. An aerosol provision system according to claim 7 or 8, when further dependent on any of claims 4-6, wherein the first channel is located outside of the cavity.

10. An aerosol provision system according to any of claims 7-9, wherein the first channel and the cavity are separated by at least one partitioning member from the aerosol provision system.

11. An aerosol provision system according to any preceding claim, wherein the first reservoir comprises a sealing member for sealing a portion of the first reservoir.

12. An aerosol provision system according to claim 11, wherein the first reservoir extends between a first end and a second end, and wherein the sealing member defines at least one of the first end and the second end of the first reservoir.

13. An aerosol provision system according to any preceding claim, wherein the first reservoir comprises a viewing means for allowing a user to observe a quantity or level of aerosolisable material inside the first reservoir, optionally wherein the viewing means comprises a transparent or translucent portion from the first reservoir.

14. An aerosol provision system according to any preceding claim, wherein the at least one air inlet comprises a plurality of air inlets.

15. An aerosol provision system according to any preceding claim, wherein the aerosol provision system further comprises a mouthpiece for receiving aerosolisable material which is vaporised by the vaporiser.
